# EUROPEAN PATENT SPECIFICATION

(11) **EP 1 774 989 B1**
(45) Date of publication and mention of the grant of the patent: **01.05.2013**
(21) Application number: 06255209.6
(22) Date of filing: 10.10.2006
(51) Int. Cl.: A61N 7/00, A61N 7/02, A61K 41/00, A61M 37/00, A61K 9/00

(54) **Treatment of cancer with high intensity focused ultrasound and chemotherapy**
Krebsbehandlung mit Hochenergieultraschall und Chemotherapie
Traitement du cancer par ultrasons haute énergie et par chimiothérapie

(30) Priority: 13.10.2005 US 726254 P; 27.07.2006 US 494300
(43) Date of publication of application: 18.04.2007
(73) Proprietor: UST Inc., Seattle, Washington 98122-5720 (US)
(72) Inventor: Larson, Eugene A., Lummi Island Washington 98262 (US); Kaminski, Perry W., Stehekin Washington 98852 (US)
(74) Representative: Gill, Stephen Charles

(56) References cited:
- WO-A-99/22652
- WO-A1-2005/002671
- US-A1- 2004 002 657
- US-A1- 2004 082 857
- US-A1- 2004 187 876
- US-A1- 2005 043 726
- US-B1- 6 308 714
- KATO M ET AL: "Mechanism of anti-tumor effect of combination of bleomycin and shock waves." JAPANESE JOURNAL OF CANCER RESEARCH : GANN OCT 2000, vol. 91, no. 10, October 2000 (2000-10), pages 1065-1072, XP002431120 ISSN: 0910-5050

## Description

The present invention describes a cancer chemotherapy drug for use in a method for treating a tumor as well as a margin of tissue surrounding the tumor with both High Intensity Focused Ultrasound (HIFU) and chemotherapy. The therapy provided combines the ability of HIFU to induce coagulative necrosis with the synergistic effects of ultrasound and chemotherapy, generally described as sonoporation. This approach is intended to treat the tumor and/or a margin surrounding the tumor to prevent the local recurrence of cancer due to cancer cells remaining in adjacent or nearby tissue.

Improved imaging technology and adoption of earlier and repeated mammography result in many breast cancers being diagnosed when tumors are one centimeter diameter or smaller, or are confined to the breast milk duct. The preferred treatment for these small cancers is to surgically remove the tumor along with an additional centimeter of tissue surrounding the tumor margin. This procedure is termed a 'breast lumpectomy,' which is currently performed on 100,000+ women each year within the U.S.

The National Surgical Adjuvant Breast and Bowel Project in Pittsburgh, PA indicated that women who had surgical 'lumpectomy' for breast tumors measuring one centimeter diameter or smaller, followed by radiation treatment of the breast, have approximately half the recurrence of "same breast" cancer as women treated with chemotherapy after lumpectomy (16.5% vs. 9.3%). Combining systemic chemotherapy with radiation therapy post lumpectomy further reduces same breast cancer (to 2.8%). There is no improvement in mortality from radiation treatments to the breast.

The cure rate of ductal carcinoma *in situ* (DCIS) treatment with lumpectomy is high if the margin of tissue around the tumor is cancer-free

Most of the 100,000+ women receiving lumpectomies are subsequently treated with chemotherapy and radiation. This combination post surgery is usually delayed until the pathology specimens have been evaluated, and in 30-40% of the lumpectomies, additional surgery or 'second lumpectomy' is performed to achieve 'clean' surgical margins free of malignant cells.

Post lumpectomy radiation therapy is either delivered from a source external to the breast, such as with a linear accelerator, or with partial breast irradiation whereby radiation is delivered from inside the breast by inserting radioactive material.

External radiation, by far the more common procedure, is typically given every weekday for three to six weeks, sometimes followed by a booster dose of five to ten treatments. In addition to the large block of time, travel and expense, radiation therapy has significant short and long term side effects, including fatigue; damage to the skin including blistering, peeling and color changes; fullness; stiffness and swelling of the breast. There may be permanent size, color and texture changes to the breast and long term damage to the heart and lungs with associated morbidity and mortality from radiation therapy. For younger women, breast radiation may permanently prevent nursing.

Internal radiation, an emerging therapy, requires either the permanent implantation of radioactive pellets or administration of daily exposures of the breast to radioactive pellets inserted into the lumpectomy through either a balloon or numerous catheters, both of which are placed into the surgical cavity.

For certain types of DCIS called "estrogen-receptor-positive DCIS", the drug tamoxifen has been used to treat early stage breast cancer after primary treatment such as lumpectomy.

With respect to brain cancer, there are approximately 17,000 primary brain tumors diagnosed in the United States each year, approximately 60% are Gliomas. Glioblastoma multiforme (GBM) is the most common and highest degree of malignancy, primarily affecting adults and most often located in the cerebral hemisphere. No significant advances have been made during the past 25 years in the treatment of GBMs and most patients die within three months without therapy. Current therapy includes surgical resection of the tumor, radiation therapy and chemotherapy which can extend life up to one year.

The treatment of brain cancer and breast cancer is somewhat similar in that the tumor is removed, followed by radiation and chemotherapy.

Tumors of the head and neck are often visible and somewhat accessible to the surgeon; however, the surgical removal of these tumors results in significant morbidity and reduction in the quality of life. Removal of tumors from the tongue can significantly affect verbal communication as well as the ability to chew and swallow. The treatment described here would be applicable to tumors of the head and neck.

Chemotherapy agents such as bleomycin, gemcitabine and oxalate platinum are known to be effective against recurrence of cancer. However, many of these drugs are toxic when given systemically. Additionally, ultrasound devices have been suggested for injecting drugs into tissue in the same manner as injecting with a hypodermic needle. In this approach, however, the ultrasound drives the drug into the tissue but there is no change in the amount of drugs that are taken-up by the cells.

It has been demonstrated that ultrasound, assisted by small encapsulated microbubbles (Ultrasound Contrast Agents--UCAs), temporarily opens cell membranes, thus allowing the introduction of macromolecules and drugs. This effect is called "sonoporation". Sonoporation can potentiate drug delivery by creating transient non-lethal perforations in cell membranes to aid ingress of large molecules and particles into the cells. Cavitation of microbubbles in capillary beds also increases capillary permeability, which improves local access of the released therapeutic agent. If cavitation nuclei are absent, the intensity of the ultrasound required for membrane permeabilization is relatively high (intensities in excess of 10 W/cm²), substantially beyond that permitted for imaging, but when microbubbles are present, this necessary intensity is greatly reduced (less than 1 W/cm²).

Although the exact mechanisms through which these drugs are transported through the cell membrane is still unknown, it is generally believed that the acoustic field causes microbubbles near the membrane surface to oscillate violently enough so that the induced shear stresses in the vicinity of the oscillating microbubble are sufficient to open gaps in the membrane. Although the transfer efficiency of sonoporation used *in vitro* and *in vivo* was found to be relatively low, it has also been shown to be as effective as electroporation in inducing the uptake of drugs into cells. See for example, Pepe J. et al., "Experimental comparison of sonoporation and electroporation in cell transfection applications", Acoustics Research Letters Online; 2004, 10.1121/1.1652111. Although UCAs greatly facilitate the sonoporation process, it is not necessary to include the UCAs along with the injected drug. What are required are cavitation nuclei-which are essentially what the UCAs provide. By agitating the fluid containing the drug, one can add, if desired, unstablized cavitation nuclei that would also facilitate sonoporation.

US 6 308 714 proposes an apparatus and method for enhancing the action of anti-cancer agents, which includes the introduction of the anti-cancer agent into or proximate a tumour within a body and thereafter introducing ultrasonic energy sufficient to increase the anti-cancer activity without significant heating of the tumour or surrounding tissue.

It has been recently demonstrated that HIFU can rapidly induce cauterization of bleeding vessels and associated tissue such that the bleeding is stopped. This use of HIFU to produce cauterization is called "acoustic hemostasis" and has considerable scientific documentation such as in Vaezy S. et al., "Liver Hemostasis using High Intensity Ultrasound: Repair and Healing", J. Ultrasound in Medicine, 2004, 23, 217-225, or Martin R.W. et al., "Water-cooled, high-intensity ultrasound surgical applicators with frequency tracking", IEEE Trans Ultrason Ferroelectr Freq Control , 2003, 50: 1305-1317. US 2004/0187816 like WO 2005/002671 A1, which is considered as closest prior art document for the invention, proposes an acoustic probe for treating cancerous tissue, the probe including an acoustic transmitter for transmitting an acoustic signal which causes damage of cancerous cells.

The in vivo activation of heat-sensitive trans-gene by HIFU for the noninvasive treatment of solid cancer tumors has been recently reported in Liu et al., "High intensity focused ultrasound-induced gene activation in solid tumors", J. Acoust. Soc. Am., 120 (1), July 2006, pp. 492-501.

The present invention describes a cancer chemotherapy drug for use in a method, using high intensity ultrasound, to treat benign and malignant tumor cells. The method comprises the use of (1) HIFU, to treat the major portion of the tumor (e.g. the primary tumor and a suitable margin surrounding the tumor) without surgical resection by inducing coagulative necrosis of the tumor cells by elevated temperature, and (2) sonoporation, though which the uptake of a locally-delivered chemotherapy agent into tumor cells is enhanced by the use of ultrasound to treat those tumor cells not destroyed by direct HIFU therapy. The present invention permits the direct destruction of tissue (ablation) of the tumor and its margin as well as the indirect destruction of tumor cells lying outside the margin, all of which can be accomplished by one or more ultrasound apparatuses used during therapy.

A first aspect of the invention provides a cancer chemotherapy drug for use in performing tumour therapy according to claim 1. A second aspect of the invention provides a cancer chemotherapy drug for use in performing tumour therapy according to claim 2.

The chemotherapy agent may, for example, be bleomycin, which is a glycosylated linear nonribosonial peptide antibiotic. When used as an "anti-cancer" or "anti neoplastic" agent, the mechanism of action of bleomycin is by induction of DNA strand break.

The present invention also relates to the use of HIFU to induce acoustic hemostasis and coagulative necrosis of the margin of tissue immediately surrounding a region of surgical resection, such as a lumpectomy, both of which can be accomplished by a single apparatus.

The ultrasound probe comprises one or more transducers. The or each transducer may be positionable on, in proximity to or within a cancerous mass of tissue. The or each transducer may be capable of delivering sufficient levels of acoustic energy to (a) induce coagulative necrosis of a region of the tissue surrounding the transducer, and optionally to (b) induce sonoporation of a chemotherapy agent into cancer cells in the tumor and in the margins of tissue adjacent the necrosis region of tissue.

The or each transducer is capable of delivering acoustic energy with an intensity of at least 100 Watts/cm². For example, in some embodiments the delivered acoustic energy may have an intensity in the range 100 to 1000 Watts/cm². However, in other embodiments, the delivered acoustic energy may have an intensity of at least 1000 Watts/cm².

### Brief Description of the Drawings

Figures 1A, 1B, and 1C show a miniature hand-held surgical instrument composed of a transducer for providing (1) HIFU for direct tumor destruction, as well as (2) low intensity pulsed plane-wave ultrasound for induction of sonoporation and enhanced take-up of a chemotherapy agent for indirect tumor cell destruction.
Figures 2A, 2B and 2C show several embodiments of transducers and arrangement of piezoelectric elements 20 that could be used to produce thermal ablation of adjacent tissue after removal of a tumor and to enable sonoporation.
Figure 3 illustrates a hydrogel sleeve 32 positioned over a transducer 31 (such as that depicted in Figure 2A) which may be used to size the transducer to the surgical cavity or wound.

Details of the present invention will be discussed with reference to particular embodiments and the accompanying drawings which are intended to represent the present invention by way of example only.

The present invention typically uses a surgical probe which emits ultrasound to uniformly cauterize (or destroy by heat) tumor tissue directly, when the ultrasound is delivered at high intensities, and indirectly, through enhanced cell uptake of a chemotherapy agent, when the ultrasound is delivered at low intensities. The invention also provides an approach of applying high intensity ultrasound with sonoporation with a local chemotherapeutic drug to the region surrounding a surgical procedure, such as a breast lumpectomy or brain tumor, thereby potentially eliminating the need for "second lumpectomies" due to tumor cells in the surgical margin, potentially eliminating post lumpectomy radiation treatment, and reducing or eliminating remaining tumors in the margin of excised tumors. Also, the invention can simultaneously provide hemostasis through cautery to terminate or prevent bleeding of the surgical wound.

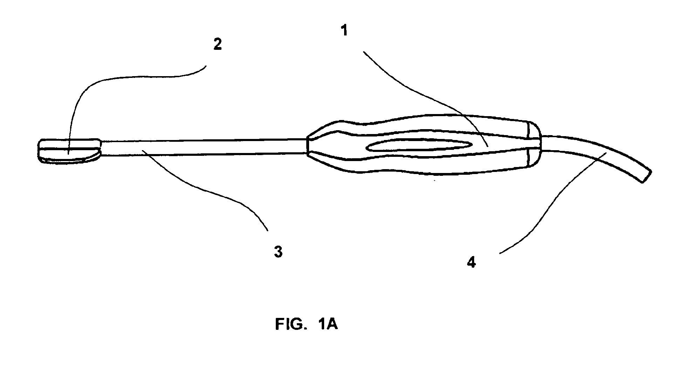

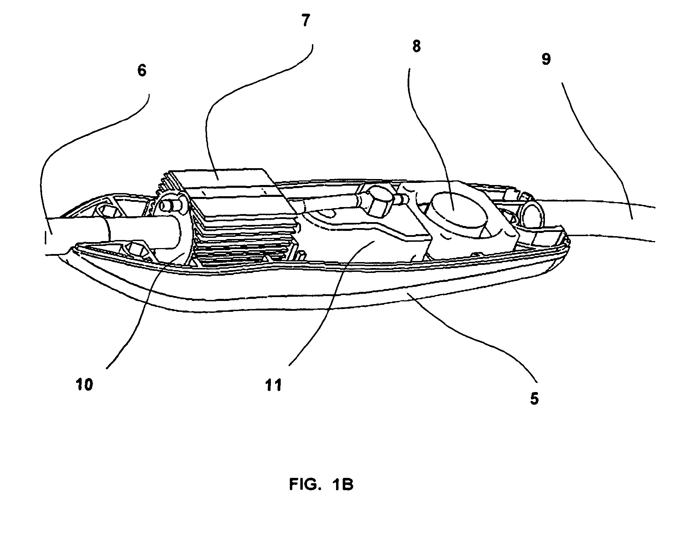

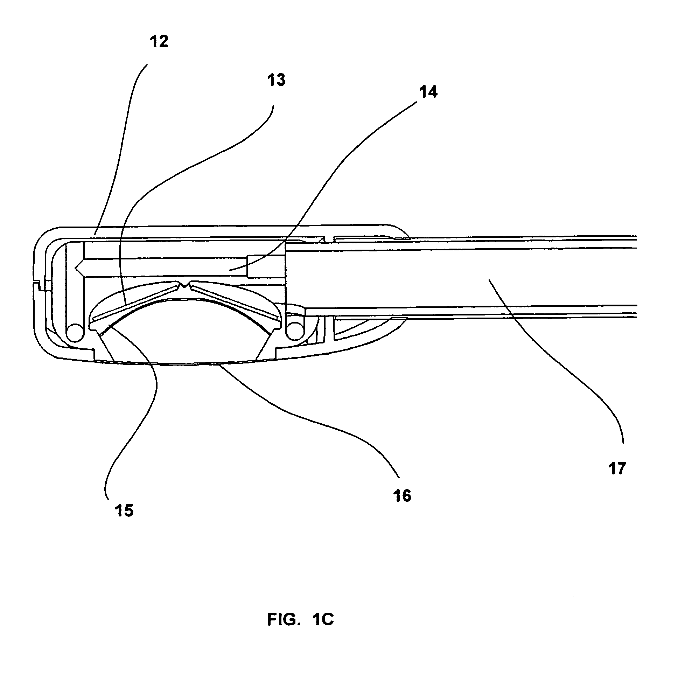

Figures 1A, 1B, 1C illustrate a miniature hand-held HIFU device that is capable of delivering HIFU to various tumor sites that are difficult to access with conventional surgical instruments.

A preferred embodiment of the hand held probe as shown in Figure 1A consists of a handle 1 connected to a slender malleable shaft 3, a piezoelectric transducer module 2 at the distal end and a connecting cable 4 at the proximal end.

Figure 1B shows the components within the handle portion 1 of Figure 1A including a handle housing 5 showing the distal end of the connecting cable 9, the proximal end of the shaft 6, which is directly connected to a fluid chamber and bubble trap 10 surrounded by a heatsink 7. Cooling fluid is circulated by means of a fluid pump 11. Air is circulated within the housing 5 by means of a fan 8.

Figure 1C shows the operative components of the transducer module 2 of Figure 1A. The transducer module consists of a housing 12 connected to the distal end of a shaft 17. Within the housing 12 is a heatsink with channels 14 through which cooling fluid is circulated. Ultrasound energy is generated by piezoelectric ceramic elements 13, focused through a lens 15 and exits through an acoustically transparent membrane 16.

In addition, the hand-held HIFU device can be used, together with the uptake of a chemotherapy agent by sonoporation, to destroy tumor cells in the region of interest that are inaccessible or missed by direct application of the ablative HIFU. Figure 1 is a drawing of a unique miniature hand-held HIFU probe. This probe is just one example of a variety of HIFU-delivery devices that could be used to produce both HIFU and sonoporation, either as a single unit, or as separate units.

A procedure for this treatment would be as follows:
(1) The bleomycin, or a similar chemotherapy agent, alone or with stabilized microbubbles, or agitated PBS, would be injected with many small needles into the tumor, including a significant margin surrounding the tumor;
(2) a HIFU probe, similar to the one shown in Fig. 1, or a modified version of this probe, would be used to deliver *short HIFU pulses* in a *plane-wave* manner, to activate the bubbles, induce transient permeability of the cell membranes, and thus permit bleomycin to be taken up by the tumor cells;
(3) the HIFU probe shown in Fig. 1, a modified version of this probe, or a separate probe, would then be used, driven in a *continuous-wave* manner, to induce thermal lesions in the tumor with HIFU.

Variations of the procedure described above may include:
(1) Because the miniature HIFU probe described in Fig. 1 delivers focused energy, it is likely that the lower ultrasound intensity, delivered in a continuous mode outside the focus, will be sufficient to initiate sonoporation, without the necessity of applying pulsed, plane wave ultrasound. In this embodiment, the tumor cells would be destroyed at their primary site by coagulative necrosis, and those in the margin would be destroyed by uptake of the chemotherapeutic drug by sonoporation;
(2) in another embodiment, the probe tip would have two transducers, mounted back-to-back (e.g. a mallet-shaped arrangement with the transducers positioned 180 degrees apart). One transducer would have a lens that would focus the ultrasound energy to induce thermal necrosis, while the other transducer would emit plane waves that would be used to induce sonoporation. This two-transducer system would require only one probe and one control module; and,
(3) in another embodiment, separate generators and/or probes would be used to affect sonoporation followed by HIFU tumor ablation.

The procedure described here has a number of applications. For example, tumors of the head and neck are often visible and somewhat accessible to the surgeon; however, the surgical removal of these tumors results in significant morbidity and reduction in the quality of life. Removal of tumors from the tongue can significantly affect verbal communication as well as the ability to chew and swallow. Metastatic breast cancer can result in numerous tumors to the chest wall, and surgical removal is often an undesirable option. No significant advances have been made during the past 25 years in the treatment of Glioblastomas and other brain tumor; consequently, most patients die within three months without therapy. Because HIFU ablation of tumors typically results in the formation of fibrotic (scar) tissue, a means to destroy tumors without surgery is a desirable clinical goal. The addition of ultrasound enhanced local chemotherapy to HIFU ablation reduces morbidity and local recurrence. The present invention can provide a non-surgical option to the successful treatment of many conditions involving benign and malignant tumors.

A specific example is the treatment of malignant breast cancer. Over 275,000 cases of breast cancer are diagnosed annually in the United States. Approximately 200,000 of these cases are invasive and the balance is carcinoma *in situ,* of which over 75% is ductal carcinoma *in situ* (DCIS).

A preferred embodiment , as illustrated in Figures 1A, 1B and 1C, shows a miniature hand-held probe that can deliver ultrasound at sufficiently high intensities so that rapid thermal denaturation of tissues in the region of interest can be accomplished. This probe has self-contained thermal cooling of the transducer, a deformable handle so that hard-to-access region of the body can be acquired, a transducer, or combination of transducers, capable of delivering HIFU for thermal therapy, as well as low intensity plane waves for induction of sonoporation and enhanced chemotherapy agent uptake. The probe can be use either to destroy a tumor directly, without surgical intervention, or to treat the region surrounding a surgically removed tumor, such as after a lumpectomy.

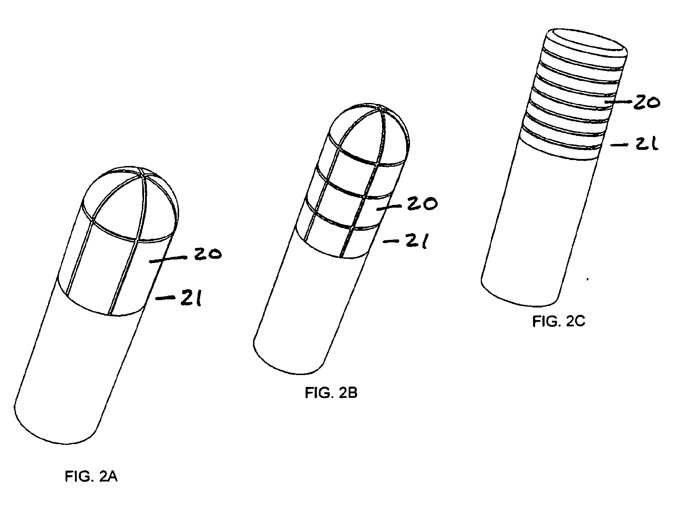

Another embodiment, as illustrated in Figures 2A, 2B and 2C, preferably comprises a transducer having a cylindrical ultrasound transducer portion 21, divided into electrically isolated segments 20 of equal electrical impedance and a spherical end transducer portion which is also divided into segments. The transducer may be supplied in multiple sizes and shapes to address the range of surgical cavity configurations.

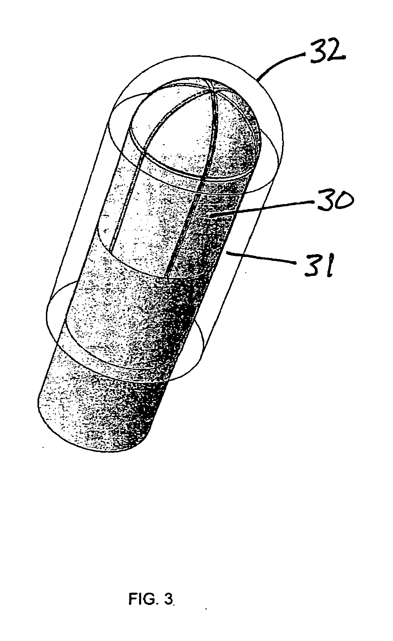

The transducer can be used with or without an acoustic sizing cap 32, as illustrated in Figure 3 surrounding the transducer body 31 and the piezoelectric elements 30. The acoustic sizing cap can be constructed of *in vivo* biocompatible hydrogels, such as those described in U.S. Patent No. 6,039,694 to Larson et al., to accommodate the surgical cavity.

In the embodiment of Figures 2A and 2C, one or more segments 20 of the transducer can be pulsed, or "activated" in a timed sequence. This feature facilitates high power pulsing without necessarily requiring significant power to pulse the entire transducer at once.

One or more probes (each having one or more transducers) cause connected to a control module. The control module includes one or more microprocessors, a frequency generator, power amplifier, and associated electronics. The control module is preferably capable of providing electrical power to drive the probes at various levels of acoustic output and delivering various forms of electrical drive to the ultrasound probes, such as continuous wave drive, pulsed wave drive, with variable pulse lengths extending from 1 millisecond pulse length to continuous wave. Preferably, the control module is capable of recognizing the ultrasound probes connected thereto through one or more approaches, such as an embedded microprocessor that has unique information about the probe or probes, a combination of resistors or a bar code. The control module also preferably comprises means (e.g. one or more pumps) to supply cooling fluids to the ultrasound probes and includes the capability of monitoring the temperature of the transducer as well as other sites within the probe itself.

Yet another embodiment utilizes High Intensity Focused Ultrasound (HIFU) to focus sound in a line focus which is then mechanically rotated in a cylindrical tube or structure to produce a continuous field of cautery and/or sonoporation. The distal end of the tube can include a spherical transducer, or an extension of the rotating line focus, to produce complete coverage of the surgical margin. A HIFU transducer configuration for producing a line focus for manually cauterizing tissue (surgeon moving the probe over an area) during surgery is described in U.S. Patent No. 6,432,067 to Martin et al.

The cylindrical structure in which the line focus transducer rotates can be constructed of many acoustic transducer materials, including low attenuation plastics and epoxies. Of particular interest are in vivo biocompatible hydrogels, such as those described in U.S. Patent No. 6,039,694.

In another embodiment, the transducer may be an ultrasound transducer, shaped into a sphere (with an access port for electrical connections), capable of radiating into 360 stereradians, so as to insonify for ablation and/or sonoporation all the tissue surrounding a removed tumor. A preferred transducer of this embodiment is a single-element piezoelectric ceramic transducer, but it is also envisioned that the transducer may be segmented into various parts so as to lower power requirements. Single-element transducers could be made in different sizes to accommodate different sizes of the void in the breast or brain produced by the removed tumor.

An additional embodiment comprises a transducer 20 having a cylindrical shape as shown in Figure 2B and including a plurality of ringed segments 20. By driving these ringed segments 20 with different electrical powers, it is possible to shape the insonified region to accommodate the individual requirements of a particular case. For example, if the tumor that was removed was close to the chest wall (in the case of breast cancer) or the body surface, one would want to shape the field so as not to damage the chest wall or sensitive skin tissue, resulting in a painful burn. In this embodiment, a balloon may enclose the transducer surface and this balloon may be filled with water or some other low-acoustic-absorption liquid. This balloon serves a variety of purposes, including (a) as a coupling medium between the transducer and the breast tissue, (b) as a means for compensating for different tissue voids within the breast, and (c) as a cooling fluid for removing heat from the transducer.

In those cases in which the tumor was close to the surface of the skin, and the desired margin was to be placed nearly to the skin surface, a thermal sink, such as cold water could be placed on the skin's surface, thus preventing damage to the dermis.

When high intensity ultrasound is applied to tissue, thermal effects can be introduced if the frequency of the ultrasound is sufficiently high. For example, frequencies in the range of 1-20 MHz can be generated by small, piezoelectric ceramic transducers. The absorption of these high frequencies by tissue results can result in a rapid temperature rise within the tissue. Piezoelectric ceramic transducers can generate acoustic intensities on their surface in the range of a few tens of Watts/cm². Thus, for a transducer of 1 cm surface area, operating at a frequency of 1 MHz, temperature elevations sufficient to induce coagulative necrosis of tissue can be accomplished in a few seconds, and the time required for the complete necrosis of a 1 cm margin around a 1 cm void in the breast is on the order of a few minutes.

Ultrasound has an advantage over other forms of therapy because of the small wavelength and the ability to direct the acoustic field, and thus the thermally ablated region, to be shaped much more readily than other thermal therapies. The inventive method provides for the delivery of high intensity ultrasound at sufficient power to cauterize or ablate tissue and to affect sonoporation of a local chemotherapeutic drug around the entire margin of a surgical cavity in a manner that does not require medical personnel to aim, or in some cases even move, the ultrasound device.

Alternatively, the application of HIFU and sonoporation may follow a surgical procedure. In this embodiment, the acoustic probe would be used to apply HIFU to the region of tissue surrounding a lumpectomy or other surgical resection of a tumor mass. Because the HIFU is able to induce acoustic hemostasis as well as coagulative necrosis, the application of the HIFU to the margin of a resected tumor would have a dual purpose: (1) It would stop bleeding, and (2) it would destroy any remaining tumor cells in the tissue margin not removed by surgery. In this embodiment, the treatment of the surrounding tissue by sonoporation and enhanced uptake of a chemotherapy agent would be an additional option. In a variation of this procedure, a chemotherapy agent would be injected into the margin and HIFU only applied. The tissue located outside the region of thermally-induced coagulative necrosis would experience sufficient levels of ultrasound energy to induce cell permeability and thus enhance the effect of the chemotherapy agent. Ultrasound enhanced local chemotherapeutic drug delivery permits much higher levels of cell uptake of drug than be accomplished with systemic chemotherapy.

As an example, the following approach is proposed for the post-operative treatment of breast and brain cancer (A similar approach would apply to cancer of other organs).
1. After the tumor and a tumor margin is surgically removed, a chemotherapy agent may be locally injected into the margin surrounding the tumor to a depth beyond that which is intended to be cauterized (ablated or destroyed) by the subsequent ultrasound treatment.
2. An ultrasound therapy transducer is inserted into the void produced by the surgery. Using an acceptable approach, such as the expanding of a balloon containing an acoustically transparent fluid, a suitable hydrogel, gel or fluid acoustic coupling agent, or by sizing the probe to the surgical cavity the therapy transducer would be coupled to the tissue.
3. Ultrasound energy radiating beyond the coagulative necrosis margin of tissue, specifically where the chemotherapeutic agent had been injected in Step 1, causes an exponential increase in cell permeability for the chemotherapeutic agent.
4. After a sufficient pause for the injected chemotherapeutic agent to diffuse through the tissue, the electrical supply to the transducer is engaged and the transducer will radiate ultrasound at an intensity and a frequency suitable for producing well controlled coagulative necrosis of a specific margin of tissue surrounding the surgical void.

It is noted that the method described herein is not specific to any one form of cancer. In particular, the present invention would have direct application to cancer of, at least, the breast, liver, pancreas, kidney, spleen, stomach, and cancers of the brain, such as infiltrative astrocytoma, pilocytic astrocytoma, oligodendroglioma, mixed oligoastrocytoma, glioblastoma multiforme (GBM), ependymoma, medulloblastoma, and primitive neural cell meningioma. In most of these cancers, it is desired to ablate or otherwise treat the infiltration of the cancer, and in many cases, removal of significant margins surrounding the primary tumor is contra-indicated.

While the invention has been described with reference to preferred embodiments it is to be understood that the invention is not limited to the particulars thereof. The present invention is intended to include modifications which would be apparent to those skilled in the art to which the subject matter pertains.

## Claims

1. A cancer chemotherapy drug which is bleomycin, gemcitabine or oxaliplatin for use in performing tumour therapy on a patient, the tumour therapy comprising injecting the drug directly into the tumor and a margin surrounding the tumor, activating a low intensity ultrasound transducer to induce sonoporation and enhanced uptake of the drug, and activating a high intensity ultrasound transducer to induce coagulative necrosis of the tumor and a margin surrounding the tumor, wherein the high intensity ultrasound transducer delivers acoustic energy with an intensity of at least 100 Watts/ cm².

2. A cancer chemotherapy drug which is bleomycin, gemcitabine or oxaliplatin for use in performing tumour therapy on a patient, the tumour therapy comprising injecting the drug directly into the tumor and a margin surrounding the tumor, and activating a high intensity ultrasound transducer in a continuous wave manner to induce coagulative necrosis of the tumor and a margin surrounding the tumor as well as to effect enhanced uptake of the drug into tumor cells in which necrosis was not induced by inducing sonoporation of the drug into said cells, wherein the high intensity ultrasound transducer delivers acoustic energy with an intensity of at least 100 Watts/ cm².

## Patentansprüche

1. Krebs-Chemotherapeutikum, das Bleomycin, Gemcitabin oder Oxaliplatin ist, zur Verwendung bei der Durchführung einer Tumortherapie an einem Patienten, wobei die Tumortherapie das Injizieren des Arzneimittels direkt in den Tumor und in einen Randbereich um den Tumor, das Aktivieren eines Ultraschallwandlers mit niedriger Intensität zur Induktion von Sonoporation und erhöhter Aufnahme des Arzneimittels und das Aktivieren eines Ultraschallwandlers mit hoher Intensität zur Induktion von koagulativer Nekrose des Tumors und eines Randbereichs um den Tumor umfasst, wobei der Ultraschallwandler mit hoher Intensität akustische Energie mit einer Intensität von zumindest 100 Watt/cm² zuführt.

2. Krebs-Chemotherapeutikum, das Bleomycin, Gemcitabin oder Oxaliplatin ist, zur Verwendung bei der Durchführung einer Tumortherapie an einem Patienten, wobei die Tumortherapie das Injizieren des Arzneimittels direkt in den Tumor und in einen Randbereich um den Tumor und das Aktivieren eines Ultraschallwandlers mit hoher Intensität in einem Dauerschallmodus zur Induktion von koagulativer Nekrose des Tumors und eines Randbereichs um den Tumor sowie zur Herbeiführung einer erhöhten Aufnahme des Arzneimittels durch die Tumorzellen, in denen keine Nekrose induziert wurde, durch die Induktion der Sonoporation des Arzneimittels in die Zellen umfasst, wobei der Ultraschallwandler mit hoher Intensität akustische Energie mit einer Intensität von zumindest 100 Watt/cm² zuführt.

## Revendications

1. Médicament de chimiothérapie du cancer qui est la bléomycine, gemcitabine ou oxaliplatine pour utilisation dans l'exécution d'une thérapie de la tumeur sur un patient, la thérapie de la tumeur comprenant l'injection du médicament directement dans la tumeur et dans une marge entourant la tumeur, à activer un transducteur ultrasonique de faible intensité pour induire une sonoporation et pour renforcer l'absorption du médicament, et à activer un transducteur ultrasonique de haute intensité pour induire une nécrose par coagulation de la tumeur et d'une marge entourant la tumeur, où le transducteur ultrasonique de haute intensité délivre une énergie acoustique d'une intensité d'au moins 100 Watts/cm2.

2. Médicament de chimiothérapie du cancer qui est la bléomycine, gemcitabine ou oxaliplatine pour utilisation dans l'exécution d'une thérapie de la tumeur sur un patient, la thérapie de la tumeur comprenant l'injection du médicament directement dans la tumeur et dans une marge entourant la tumeur, et à activer un transducteur ultrasonique de haute intensité à la manière d'une onde continue pour induire une nécrose par coagulation de la tumeur et d'une marge entourant la tumeur et à entraîner une plus grande absorption du médicament par les cellules de la tumeur dans lesquelles la nécrose n'a pas été induite en induisant la sonoporation du médicament dans lesdites cellules, où le transducteur ultrasonique de haute intensité délivre une énergie acoustique d'une intensité d'au moins 100 Watts/cm2.
